# EUROPEAN PATENT SPECIFICATION

(11) **EP 0 442 671 B1**
(45) Date of publication and mention of the grant of the patent: **07.06.1995**
(21) Application number: 91301053.4
(22) Date of filing: 11.02.1991
(51) Int. Cl.: A61K 9/52, A61K 38/00, A61K 38/08, B01J 13/02

(54) **Prolonged release microcapsules**
Mikrokapseln mit verzögerter Wirkstoffabgabe
Microcapsules à libération prolongée

(30) Priority: 13.02.1990 JP 33133/90
(43) Date of publication of application: 21.08.1991
(73) Proprietor: Takeda Chemical Industries, Ltd., Chuo-ku, Osaka (JP)
(72) Inventor: Okada, Hiroaki, Suita, Osaka 565 (JP); Inoue, Yayoi, Kyoto 615 (JP); Ogawa, Yasuaki, 77-42, Koaza-tanita, Otokuni-gun, Kyoto 618 (JP)
(74) Representative: Lewin, John Harvey

(56) References cited:
- EP-A- 0 052 510
- EP-A- 0 145 240
- EP-A- 0 190 833
- EP-A- 0 202 065
- EP-A- 0 256 726
- EP-A- 0 350 246

## Description

### Field of the Invention

This invention relates to microcapsules designed for sustained release of luteinizing hormone-releasing hormone (LH-RH) or an analog thereof.

### Background of the Invention

Various dosage forms have been proposed for drugs required to be administered for a prolonged period. Among them, Japanese published unexamined patent application (Toku-Kai Sho) 57-118512 and its corresponding EP-A-0052510 disclose preparation of microcapsules by a phase separation method using a coacervation agent such as a mineral oil or a vegetable oil. Toku-Kai Sho 60-100516 (the corresponding U.S. Patent Nos. 4652441 and 4711782), 62-201816 (the corresponding EP-A-0190833) and 63-41416 disclose methods of preparing microcapsules by means of in-water drying. According to these methods, drugs can be efficiently incorporated into microcapsules to give desirable microcapsules with less initial release.

In the case of administering a drug in the form of microcapsules, requirements for microcapsules having high dependency on interaction with functions of living body are diversified into a variety of phases. Since the matter is concerned with medicines, microcapsules capable of satisfying those various requirements as far as possible have been desired.

There are many reports on microcapsules comprising a water-soluble drug using a biodegradable polymer. However, in the case of using a water-soluble drug, especially a luteinizing hormone-releasing hormone (LH-RH) or an analog thereof having a relatively large molecular weight, the diffusion of the drug thus encapsulated into the polymer is low, and, therefore, the drug is not released at the initial stage until the decomposition or impregnating of the polymer proceeds, and, adversely, a large burst at the initial stage cannot be avoided depending on the method of preparation, thus there are often the cases that practical difficulties occur when used as medicines. Especially, in sustained release pharmaceutical compositions over an extended period of time, constant release of the drug with higher accuracy is an important requirement, but no microcapsules satisfying those requirements have been known.

### Objects of the Invention

In view of these circumstances, the present inventors have conducted intensive studies with the purpose of developing pharmaceutical compositions designed for sustained release of a luteinizing hormone-releasing hormone (LH-RH) or an analog thereof over an extended period of time. As a result, the present inventors found that, by preparing microcapsules using suitably selected polylactic acid of a limited molecular weight or lactic acid-glycolic acid (100/0 to 90/10), microcapsules having continuous excellent releasability for a long time were obtained. Further research work based on this finding has now led to completion of the present invention.

### Summary of the Invention

More specifically, the present invention provides a microcapsule designed for zero order release of luteinizing hormone-releasing hormone (LH-RH) or an analog thereof over a period of at least two months, which is produced by preparing a water-in-oil emulsion comprising an inner aqueous phase containing 20 to 70% (w/w) of said LH-RH or analog, in the absence of a drug-retaining substance, and an oil phase containing a copolymer or homopolymer having a weight-average molecular weight of 7,000 to 30,000, the copolymer or homopolymer having a molar composition ratio of lactic acid/glycolic acid of 90/10 to 100/0, and then subjecting said water-in oil emulsion to microencapsulation.

The present invention also provides a process for preparing a microcapsule designed for zero order release of luteinizing hormone-releasing hormone (LH-RH) or an analog thereof over a period of at least two months, which comprises preparing a water-in-oil emulsion comprising an inner aqueous phase containing 20 to 70% (w/w) of the said LH-RH or analog in the absence of a drug-retaining substance, and an oil phase containing a copolymer or homopolymer having a weight average molecular weight of 7,000 to 30,000, the copolymer or homopolymer having a molar composition ratio of lactic acid/glycolic acid of 90/10 to 100/0, and then subjecting said water-in-oil emulsion to an in-water drying or phase-separation method.

### Detailed Description of the Invention

Luteinizing hormone-releasing hormone (LH-RH) and its analogs include, for example, substances having LH-RH like activity [cf. U.S. Patents Nos. 3,853,837, 4,008,209, 3,972,859 and 4,234,571, British Patent No. 1,423,083, Proceedings of the National Academy of Sciences of the United States of America, Volume 78, pages 6509-6512 (1981)] and LH-RH antagonists (cf. U.S. Patents Nos. 4,086,219, 4,124,577, 4,253,997 and 4,317,815).

Especially, in a microcapsule comprising a luteinizing hormone-releasing hormone (LH-RH) or an analog thereof which is water-soluble and has a molecular weight of 1,000 or more, [e.g. TAP-144 expressed by (pyr)Glu-His-Trp-Ser-Tyr-D-Leu-Leu-Arg-ProNHC₂H₅, or LH-RH antagonist expressed by (pyr)Glu-His-Trp-Ser-Tyr-Trp-Leu-Arg-Pro-GlyNHC₂H₅], continuous sustained release is performed advantageously over a prolonged period of time.

These luteinizing hormone-releasing hormones (LH-RH) or analogs thereof are used in amounts selected largely depending on the kind of peptide, desired pharmacological effects and duration of the effects, among others, and the amount ranges from about 0.01 mg to 5 g, more preferably, from 0.1 mg to 2 g, as the dosage of microcapsules. The concentration in a microcapsule depends on the physico-chemical properties of the drug, and it is selected within the range of about O.01% to about 50% (w/w), more preferably within the range of O.1% to 30% (w/w).

The concentration of said polypeptide in the inner aqueous phase of a microcapsule ranges from about 20% to 70 % (w/w), preferably 25 to 65 % (w/w), more preferably 35 to 60% (w/w) while it depends on its physico-chemical properties such as the solubility in water.

Examples of the polymer used as a release-controlling substance include copolymers or homopolymers of lactic acid/glycolic acid which has an acid residue in the molecule, is hardly soluble or insoluble in water and is biocompatible. The ratio of them depends on the period required for sustained release, and is selected from the range from 100/0 to 90/10, preferably 100/O.

As lactic acid, L-, D- and DL-lactic acid can be used, especially copolymer or homopolymer prepared by polymerization of monomer or oligomer of DL-lactic acid is advantageously utilized.

As the copolymer or homopolymer consisting of DL-lactic acid/glycolic acid, such polymers containing substantially no catalyst as obtained by polymerization in the absence of catalyst are advantageously used (cf. Toku-Kai Sho 61-285215). Polymers having a dispersion degree (ratio of weight-average molecular weight to number-average molecular weight) of 1.5 to 3.O, especially 1.5 to 2.5 are preferable.

Length of the period of continuous sustained release of microcapsules of this invention largely depends on the molecular weight of a polymer and the composition ratio of lactic acid/glycolic acid. In the case of preparing, for example, microcapsules performing continuous zero order release for at least three months, when the composition ratio of lactic acid/glycolic acid is 100/0, preferable average-weight molecular weight of a polymer ranges from 7,000 to 25,000; and when 90/10, from 7,000 to 30,000.

In the present specification, the weight-average molecular weight and the degree of dispersion mean values which are determined by means of a gel- permeation chromatography using commercially available polystyrene of standard molecular weight.

The concentration of a polymer in the oil phase when preparing microcapsules is selected from the range of about 0.5 to 90% (w/w), more preferably from the range of about 2 to 60% (w/w).

The solution (oil phase) containing the abovementioned polymer is that of the polymer dissolved in an organic solvent.

Said organic solvent may be any organic solvent which has a boiling point not higher than about 120°C and hardly miscible with water. Examples are halogenated alkanes (e.g. dichloromethane, chloroform, chloroethane, trichloroethane, carbon tetrachloride, etc.), ethyl acetate, ethyl ether, benzene, toluene, etc. These may be used in admixture of two or more.

In the present invention, microcapsules are prepared in the absence of a drug retaining substance.

As conventional methods of controlling the releasability of these microcapsules, mention is made of a method of changing the hydrolysis rate [Biomaterials Vol. 5, 237-240 (1984)] and a method comprising incorporation of a water-soluble compound into matrix of microcapsules to create aqueous channels for releasing the drug. However, the former tends to invite shortening of a long-term of release, and the latter induces only an initial burst, thus an approximate zero-order release can hardly be expected, [Chem. Pharm. Bull. Vol. 36(4) 1502-1507 (1988)]. And, in the latter case, there is a fear of occurrence of undesirable side effects due to the increase of drug in blood at the initial stage. Further, there is also a known method (Toku-Kai Sho 57-150609), which comprises having the polymerization ratio of lactic acid/glycolic acid of PLGA to improve the time of suspending the release. This method is, however, directed to increase the speed of decomposition of the polymer, which, naturally, shortens the period of lasting the release, thus there is a limit in realizing continuous release for a long period of time.

The sustained-release microcapsules of the present invention are prepared by, for example, the following method.

A luteinizing hormone-releasing hormone (LH-RH) or an analog thereof is added to water in an amount of realizing the above-mentioned concentration.

To this inner aqueous phase, there may be added a pH-adjusting agent for maintaining the stability or solubility of the luteinizing hormone-releasing hormone (LH-RH) or an analog thereof such as carbonic acid, acetic acid, oxalic acid, citric acid, phosphoric acid, hydrochloric acid, sodium hydroxide, arginine, lysine and their salts. And, there may further be added, as a stabilizer of the luteinizing hormone-releasing hormone (LH-RH) or an analog thereof albumin, gelatin, citric acid, sodium ethylenediamine tetraacetate, dextrin, sodium hydrogen sulfite or a polyol compound such as polyethylene glycol, or, as a preservation, there may be added conventionally usable ones, such as a para-hydroxybenzoic acid ester (e.g. methylparaben, propylparaben), benzyl alcohol, chlorobutanol or thimerosal.

The thus-obtained inner aqueous phase is added to a polymer-containing solution (oil phase), followed by an emulsification procedure to give a W/O type emulsion. For said emulsification procedure, a known method of effecting dispersion is employed. As the method, mention is made of, for example, the intermittent shaking method, the method using a mixer such as a propeller-shaped stirrer a turbine-shaped stirrer or the like, the colloid mill method, the homogenizer method or the ultrasonification method.

Then, the thus-prepared W/O emulsion is subjected to microencapsulation. An in-water drying or phase-separation method may be employed as a mean of microencapsulation. In the case of preparing microcapsules by the in-water drying, said W/O emulsion is further added to a third aqueous phase to give a W/O/W ternary emulsion and, thereafter, the solvent in the oil phase is evaporated off to give microcapsules.

To the external aqueous phase, there may be added an emulsifying agent. As the emulsifying agent, there may be used any one capable of forming generally a stable O/W emulsion, for example an anionic surfactant (e.g. sodium oleate, sodium stearate, sodium lauryl sulfate, etc.), a nonionic surfactant (e.g. polyoxyethylenesorbitan fatty acid ester (Tween 80, Tween 60, products of Atlas Powder Co.), a polyoxyethylene castor oil derivative (HCO-60, HCO-50, products of Nikko Chemicals), etc.), polyvinyl pyrrolidone, polyvinyl alcohol, carboxymethylcellulose, lecithin or gelatin. Such emulsifiers may be used either alone or in combination or some of them. The emulsifying agent concentration may suitably be selected within the range of about 0.01% to 20%, preferably within the range of about 0.05% to 10%.

For evaporation of the solvent from the oil phase, any of the common methods in general use is employed. The method is conducted by, for example, gradually reducing the pressure while stirring with a propeller-shaped stirrer or a magnetic stirrer, or by using a rotary evaporator while adjusting the degree of vacuum. In this case, the required time can be reduced by gradually warming the W/O/W emulsion after the progress of solidification of the polymer to a certain extent for rendering the solvent removal more complete.

The thus-produced microcapsules are collected by centrifugation or filtration, rinsed several times with distilled water to thereby remove the free luteinizing hormone-releasing hormone (LH-RH) or an analog thereof and the emulsifying agent adhering to the microcapsule surface, followed by dispersing the resultant in e.g. distilled water and by freeze-drying, which is, if necessary, warmed under reduced pressure to thereby remove the moisture in microcapsules and the solvent in the microcapsule wall more completely.

In the case of preparing microcapsules by the phase-separation method, a coacervation agent is gradually added to the said W/O emulsion under stirring to allow the polymer to precipitate and solidify.

A coacervation agent may be any solvent-miscible polymeric, mineral oil or vegetable oil compounds as exemplified by silicone oil, sesame oil, soybean oil, corn oil, cotton seed oil, coconut oil, linseed oil, mineral oils, n-hetane, n-heptane etc. These may be used as a mixture of two or more of them.

The microcapsules obtained thus above were collected by filtration and washed with, for example, heptane, repeatedly to remove the poor solvent of the polymer. Further, removal of the free drug and separation of the solvent were conducted in a manner similar to the in-water drying process. For preventing aggregation of microcapsules to one another during the washing, an agent for preventing aggregation may be added.

The microcapsules of the present invention designed for sustained-release produced by the abovementioned in-water drying process more preferably perform a stable sustained-release for a long period of time.

Dosage forms of administering microcapsules of the present invention include injections, implantations and agents absorbed through mucous membrane of rectum or uterus.

The microcapsules obtained in the above manner are sieved, when necessary after slightly crushing, to eliminate excessively large microcapsules. The average grain size of microcapsules is within the range from about 0.5 to 1000 »m, desirable and preferably within the range of about 2 to 500 »m. When the microcapsules are used as injections in the form of suspension, the grain size may be sufficient so long as it satisfies the requirements for dispersability and injectability, for example, desirably within the range of about 2 to 100 »m.

The microcapsules produced by the methods according to this invention have many advantages. For instance, they scarcely undergo aggregation or cohesion to one another during the production step. There can be obtained microcapsules which are satisfactorily spherical in shape having an optional size. The step of removing the solvent from the oil phase is easy to control, whereby the surface structure of microcapsules, which is decisive for the rate of drug release can be controlled.

The microcapsules produced by the method of this invention can be easily administered as injections and implants intramuscularly, subcutaneously, intravenously, or at an organ, joint cavity or at a lesion such as tumors. They may also be administered in various dosage forms and thus can be used as materials in preparing such dosage forms.

For instance, in making up the microcapsules according to this invention for an injection, the microcapsules according to the invention are dispersed in an aqueous medium together with a dispersing agent (e.g. Tween 80, HCO-60, carboxymethylcellulose, sodium alginate, etc.), a preservative (e.g. methylparaben, propylparaben, etc.), an isotonizing agent (e.g. sodium chloride, mannitol, sorbitol, glucose, etc.), or suspended in an aqueous medium together with a vegetable oil such as sesame oil or corn oil. Such dispersion or suspension is formulated into a practically usable sustained-release injection.

Furthermore, the above microencapsulated sustained-release injection can be converted to a more stable, sustained-release injection by adding an additional excipient (e.g. mannitol, sorbitol, lactose, glucose, etc.), redispersing the resulting mixture and effecting solidification by freeze-drying or spray drying with extemporaneous addition of distilled water for injection or some appropriate dispersing agent.

The dose of the sustained-release preparation according to this invention may vary depending on the kind and amount of the luteinizing hormone-releasing hormone (LH-RH) or an analog thereof which is the active ingredient, dosage form, duration or drug release, recipient animal (eg warm-blooded animals such as mouse, rat, rabbit, sheep, pig, cow, horse, human) and purpose of administration but should be within the range of effective dose of said active ingredient. For example, the single dose per said animal of the microcapsules can adequately be selected within the range of about 0.1 mg to 100 mg/kg body weight, preferably about 0.2 mg to 50 mg/kg body weight.

In this manner, there is obtained a pharmaceutical composition prepared in the form of microcapsules which comprises an effective but greater amount of the luteinizing hormone-releasing hormone (LH-RH) or an analog thereof as compared with the ordinary single dose and a biocompatible polymer and is capable of releasing the drug continuously over a prolonged period of time.

The sustained-release preparation according to the present invention has the following characteristics, among others: (1) Continuous sustained-release of the luteinizing hormone-releasing hormone (LH-RH) or an analog thereof can be attained in various dosage forms. In particular, where a long-term treatment with an injection is required, the desired therapeutic effects can be achieved in a stable manner by injection of the preparation once in three month, or once in six months, instead of daily administration. Thus, said preparation can achieve a sustained drug release over a longer period as compared with the conventional sustained-release preparations. (2) When the preparation in which a biodegradable polymer is used is administered in the form of an injection, such surgical operation as implantation is no more required but the preparation can be administered subcutaneously, intramuscularly, or at an organ or a lesion, with ease in quite the same manner as the ordinary suspension injections. And, there is no need for taking the matrix out from the body after completion of the drug release.

The following Reference Example and Examples illustrate the invention in further detail.

### Reference Example 1

A four-necked flask equipped with a thermometer, a condenser and an inlet of nitrogen was charged with 160 g of a 85% aqueous solution of DL-lactic acid. The solution was heated under reduced pressure for six hours in nitrogen streams at inner temperatures and pressures ranging from 105°C and 350 mmHg to 150^{o}C and 30 mmHg to remove water thus distilled. The reaction was allowed to proceed at 175^{o}C for 90 hours under reduced pressure of 3 to 5 mmHg, which was then cooled to room temperatures to give 98 g of a substantially colorless massive polymer. This polymer was dissolved in tetrahydrofuran and the weight-average molecular weight and the degree of dispersion were determined by means of a gel-permeation chromatography using commercially available polystyrene of standard molecular weight to find 17,200 and 1.89, respectively.

### Example 1

TAP-144 (400 mg) was dissolved in 0.5 ml of distilled water to give an aqueous phase. The aqueous solution was added to a solution of 4 g of poly-DL-lactic acid [Lot No. 870818, weight-average molecular weight 18,000 (microcapsule Lot No. 244, 245) and Lot No. 880622, weight-average molecular weight 18,200, dispersity 1.76 (microcapsule Lot No. 248)] in 7.5 ml of dichloromethane. The mixture was stirred in a small-size homogenizer (Polytron, product of Kinematica, Switzerland) for about 60 seconds to give a W/O emulsion. This emulsion was cooled to 15°C. This emulsion was then poured into 1,000 ml of a 0.25% aqueous solution (previously cooled at 15°C) of polyvinyl alcohol (PVA) which was stirred using a small-size homogenizer to give a W/O/W emulsion. Thereafter, dichloromethane was evaporated off, while stirring the W/O/W emulsion, to thereby solidify the inner W/O emulsion, followed by collecting thus solidified material by centrifugation.

The material was again dispersed in distilled water, which was subjected to centrifugation, followed by washing the drug and the dispersant then liberated.

Microcapsules thus collected were subjected to freeze-drying to remove the solvent and to dehydrate more completely to give powdery product. The content of the drug to be taken up in the microcapsules (Lot. 244, 245, 248) was prescribed as 9%, and the entrapped ratio was 100% or more.

These microcapsules were administered to rats (n=5) subcutaneously, then the TAP-144 remaining in the microcapsules at the injection site was determined quantitatively to measure the in vivo release rate of the drug. The results are shown in Table-1.

**Table-1**

| in-vivo release-rate | | | | | |
|---|---|---|---|---|---|
| Lot | Amount of Drug Remaining Subcutaneously (%) | | | | |
| | 1 day | 2 weeks | 4 weeks | 8 weeks | 14 weeks |
| 244 | 102.2 | 89.0 | 70.2 | 44.0 | 9.5 |
| 245 | 105.9 | 82.4 | 69.4 | 52.1 | 9.8 |
| 248 | 104.1 | 75.4 | 72.8 | 43.7 | 11.6 |

These microcapsules do not show initial burst and continuous release of TAP-144 was observed for 14 weeks, i.e. longer than 3 months, with substantially good reproducibility.

### Example 2

Similarly, TAP-144 (400 mg) was dissolved in 0.5 ml of distilled water to give an aqueous phase. Four grams of poly-DL-lactic acid having a weight-average molecular weight of 8,400 (Lot. 870304, microcapsule Lot. 312) was dissolved in 5 ml of dichloromethane to give an oil phase. The aqueous phase and the oil phase were mixed in the same manner as described above to give a W/O emulsion.

This emulsion was cooled to 13°C, which was poured into 1,000 ml of 0.25% aqueous solution of polyvinyl alcohol (PVA). The mixture was processed in the same manner as described above to give a W/O/W emulsion, which was prepared into microcapsules.

Further, 550 mg of TAP-144 was dissolved in 1 ml of distilled water. On the other hand, 4 g each of three samples of poly-DL-lactic acid (Lot No. 890717, molecular weight 14,100, dispersity 2.00 microcapsule Lot. 402; Lot No. 890720, molecular weight 17,200, dispersity 1.89, microcapsule Lot No. 405; Lot No. 890721, molecular weight: 17,500, dispersity: 1.87, microcapsule Lot No. 406) was dissolved in 7.5 ml each of dichloromethane. The above aqueous solution was added to each of the three samples dissolved in dichloromethane, followed by processing in the same manner as above to give three samples of W/O emulsion. The respective emulsions were poured into 1,000 ml each of three samples of 0.25% aqueous solution of polyvinyl alcohol previously cooled at 15^{o}C (the first one) and at 18°C (the second and third ones), which were respectively processed in the same manner as described in the forgoing to obtain microcapsules. The entrapped ratios of the drug were 101%, 113% and 103%, respectively.

Table-2 shows in vivo release rates of the drug in the respective microcapsules measured in the same manner as described above.

**Table-2**

| Lot | n | Amount of Drug Remaining Subcutaneously (%) | | | | | |
|---|---|---|---|---|---|---|---|
| | | 1 day | 1 week | 2 weeks | 8 weeks | 12 weeks | 14 weeks |
| 312 | 5 | 86.3 | 82.2 | 41.2 | 9.8 | - | - |
| 402 | 3 | 98.0 | 78.2 | 64.9 | 38.4 | 20.0 | - |
| 405 | 5 | 88.8 | 79.4 | 52.2 | 33.8 | - | 21.3 |
| 406 | 5 | 85.5 | 86.2 | 56.7 | 38.8 | - | 23.1 |

The release of the drug, after a small amount of initial release, shows a continuous long release over longer than two months. The term of release was dependent upon the hydrolysis rate of the high molecular polymer then employed.

### Example 3

Microcapsules were prepared, in the same manner as Example 1, from an aqueous phase prepared by dissolving 400 mg of TAP-144 in 0.5 ml of distilled water and an oil phase prepared by dissolving 4 g of polylactic acid - glycolic acid (90/10) [Lot No. 870320 (weight-average molecular weight : 19,000), microcapsule Lot No. 315, Lot No. 891020 (weight-average molecular weight : 13,800), microcapsule Lot No. 410]. Referring to the microcapsule Lot No. 410, an aqueous solution prepared by dissolving 550 mg of TAP-144 in 1 ml of distilled water was used as the inner aqueous phase, and the temperatures of the W/O emulsion and the external phase were adjusted to 15°C and 18°C, respectively. The entrapped ratios of the drug in these microcapsules were 106% and 100%, respectively.

These microcapsules were administered to rats subcutaneously in the same manner as described above, and their in vivo release rates of the drug were evaluated. Table-3 shows that sustained-release microcapsules for a continuous prolonged period over more than two months were obtained.

**Table-3**

| in vivo release-rate (n=5) | | | | | | | |
|---|---|---|---|---|---|---|---|
| Lot | Amount of Drug Remaining Subcutaneously (%) | | | | | | |
| | 1 day | 1 week | 2 weeks | 4 weeks | 6 weeks | 8 weeks | 10 weeks |
| 315 | 77.4 | 76.0 | 59.2 | 51.6 | 41.1 | 25.8 | - |
| 410 | 93.5 | 88.3 | 64.1 | 52.5 | 33.1 | 32.7 | 15.4 |

### Example 4

Microcapsules were prepared, in the same manner as Example 1, from an aqueous phase prepared by dissolving 280 mg of TRH (free form) in 0.25 ml of distilled water and an oil phase prepared by dissolving, in 6 ml of dichloromethane, poly-DL-lactic acid (average molecular weight 17,200, dispersity 1.89) employed in Example 2, and by adjusting the temperature of the W/O emulsion and external aqueous phase at 15°C. The entrapped ratio of the drug in the microcapsules thus obtained (Lot No. R-103) was 85.8%.

Table-4 shows that the release of the drug in thus-obtained microcapsule was such long-lasting as covering about 3 months.

**Table-4**

| Lot | Amount of Drug Remaining Subcutaneously (%) | | | | |
|---|---|---|---|---|---|
| | 1 day | 2 weeks | 4 weeks | 8 weeks | 12 weeks |
| R103 | 98.3 | 80.0 | 61.8 | 30.6 | 6.7 |

## Claims

1. A microcapsule designed for zero order release of luteinizing hormone-releasing hormone (LH-RH) or an analog thereof over a period of at least two months, which is produced by preparing a water-in-oil emulsion comprising an inner aqueous phase containing 20 to 70% (w/w) of said LH-RH or analog, in the absence of a drug-retaining substance, and an oil phase containing a copolymer or homopolymer having a weight-average molecular weight of 7,000 to 30,000, the copolymer or homopolymer having a molar composition ratio of lactic acid/glycolic acid of 90/10 to 100/0, and then subjecting said water-in oil emulsion to microencapsulation.

2. The microcapsule according to claim 1, wherein said water-in-oil emulsion is dispersed in an aqueous phase and then the resulting water/oil/water ternary emulsion is subjected to an in-water drying.

3. The microcapsule according to claim 1, wherein the LH-RH analog is water-soluble and has a molecular weight of 1,000 or more.

4. The microcapsule according to claim 1, wherein said LH-RH analog is (pyr)Glu-His-Trp-Ser-Tyr-D-Leu-Leu-Arg-ProNHC₂H₅ (TAP-144).

5. The microcapsule according to claim 1, wherein said homopolymer is a poly-lactic acid having a weight-average molecular weight of 7,000 to 25,000.

6. The microcapsule according to claim 1, wherein said inner aqueous phase contains 25 to 65% (w/w) of said LH-RH or analog.

7. The microcapsule according to claim 1, wherein said inner aqueous phase contains 35 to 60% (w/w) of said LH-RH or analog.

8. A process for preparing a microcapsule designed for zero order release of luteinizing hormone-releasing hormone (LH-RH) or an analog thereof over a period of at least two months, which comprises preparing a water-in-oil emulsion comprising an inner aqueous phase containing 20 to 70% (w/w) of the said LH-RH or analog in the absence of a drug-retaining substance, and an oil phase containing a copolymer or homopolymer having a weight average molecular weight of 7,000 to 30,000, the copolymer or homopolymer having a molar composition ratio of lactic acid/glycolic acid of 90/10 to 100/0, and then subjecting said water-in-oil emulsion to an in-water drying or phase-separation method.

9. The process according to claim 8, wherein said water-in-oil emulsion is dispersed in an aqueous phase and then the resulting water/oil/water ternary emulsion is subjected to an in-water drying.

10. The process according to claim 8, wherein the said water-in-oil emulsion is dispersed in an aqueous phase containing polyvinyl alcohol as an emulsifying agent.

11. The process according to claim 8, wherein said LH-RH analog is water-soluble and has a molecular weight of 1,000 or more.

12. The process according to claim 8, wherein said LH-RH analog is (pyr)Glu-His-Trp-Ser-Tyr-D-Leu-Leu-Arg-ProNHC₂H₅ (TAP-144).

13. The process according to claim 8, wherein said homopolymer is a poly-lactic acid having a weight-average molecular weight of 7,000 to 25,000.

14. The process according to claim 8, wherein said inner aqueous phase contains 25 to 65% (w/w) of said LH-RH or analog.

15. The process according to claim 8, wherein said inner aqueous phase contains 35 to 60% (w/w) of said LH-RH or analog.

## Patentansprüche

1. Mikrokapsel zur "nullter"-Ordnung-Freisetzung Von luteinisierendes Hormon freisetzendem Hormon (LH-RH) oder eines Analogen davon über eine Zeitspanne von zumindest zwei Monaten, die durch Bildung einer Wasser-in-Öl-Emulsion, umfassend eine innere wäßrige Phase, die 20 - 70 Gew.-% LH-RH oder Analoges ohne eine wirkstoff-zurückhaltende Substanz enthält, und eine Ölphase, die ein Copolymer oder Homopolymer mit einem durchschnittlichen Molekulargewicht von 7.000 - 30.000 enthält, wobei das Copolymer oder Homopolymer ein molares Zusammensetzungsverhältnis Milchsäure/Glykolsäure von 90/10 bis 100/0 aufweist, und Mikroeinkapselung der Wasser-in-Öl-Emulsion hergestellt wird.

2. Mikrokapsel nach Anspruch 1, worin die Wasser-in-Öl-Emulsion in einer wäßrigen Phase dispergiert und die resultierende ternäre Wasser-Öl-Wasser-Emulsion einer In-Wasser-Trocknung unterzogen wird.

3. Mikrokapsel nach Anspruch 1, worin das LH-RH-Analoge wasserlöslich ist und ein Molekulargewicht von 1.000 oder mehr aufweist.

4. Mikrokapsel nach Anspruch 1, worin das LH-RH-Analoge (pyr)Glu-His-Trp-Ser-Tyr-D-Leu-Leu-Arg-ProNHC₂H₅ (TAP-144) ist.

5. Mikrokapsel nach Anspruch 1, worin das Homopolymer eine Polymilchsäure mit einem durchschnittlichen Molekulargewicht von 7.000 - 25.000 ist.

6. Mikrokapsel nach Anspruch 1, worin die innere wäßrige Phase 25 - 65 Gew.-% LH-RH oder Analoges enthält.

7. Mikrokapsel nach Anspruch 1, worin die innere wäßrige Phase 35 - 60 Gew.-% LH-RH oder Analoges enthält.

8. Verfahren zur Herstellung einer Mikrokapsel zur "nullter"-Ordnung-Freisetzung von luteinisierendes Hormon freisetzendem Hormon (LH-RH) oder eines Analogen davon über eine Zeitspanne von zumindest zwei Monaten, das die Bildung einer Wasser-in-Öl-Emulsion, umfassend eine innere wäßrige Phase, die 20 - 70 Gew.-% LH-RH oder Analoges ohne eine wirkstoff-zurückhaltende Substanz enthält, und eine Ölphase, die ein Copolymer oder Homopolymer mit einem durchschnittlichen Molekulargewicht von 7.000 - 30.000 enthält, wobei das Copolymer oder Homopolymer ein molares Zusammensetzungsverhältnis Milchsäure/Glykolsäure von 90/10 bis 100/0 aufweist, und das Unterziehen der Wasser-in-Öl-Emulsion einer In-Wasser-Trocknung oder einem Phasentrennverfahren umfaßt.

9. Verfahren nach Anspruch 8, worin die Wasser-in-Öl-Emulsion in einer wäßrigen Phase dispergiert und die resultierende ternäre Wasser-Öl-Wasser-Emulsion einer In-Wasser-Trocknung unterzogen wird.

10. Verfahren nach Anspruch 8, worin die Wasser-in-Öl-Emulsion in einer wäßrigen Phase dispergiert wird, die Polyvinylalkohol als Emulgator enthält.

11. Verfahren nach Anspruch 8, worin das LH-RH-Analoge wasserlöslich ist und ein Molekulargewicht von 1.000 oder mehr aufweist.

12. Verfahren nach Anspruch 8, worin das LH-RH-Analoge (pyr)Glu-His-Trp-Ser-Tyr-D-Leu-Leu-Arg-ProNHC₂H₅ (TAP-144) ist.

13. Verfahren nach Anspruch 8, worin das Homopolymer eine Polymilchsäure mit einem durchschnittlichen Molekulargewicht von 7.000 - 25.000 ist.

14. Verfahren nach Anspruch 8, worin die innere wäßrige Phase 25 - 65 Gew.-% LH-RH oder Analoges enthält.

15. Verfahren nach Anspruch 8, worin die innere wäßrige Phase 35 - 60 Gew.-% LH-RH oder Analoges enthält.

## Revendications

1. Microcapsule conçue pour une libération d'ordre zéro de l'hormone libérant l'hormone lutéinisante (LH-RH) ou d'un analogue de celle-ci pendant un laps de temps d'au moins deux mois, qui est obtenue en préparant une émulsion d'eau dans l'huile comprenant une phase aqueuse interne contenant de 20 à 70% (poids/poids) de ladite LH-RH ou d'un analogue, en l'absence d'un agent de rétention du médicament, et une phase huileuse contenant un copolymère ou un homopolymère ayant un poids moléculaire moyen pondéral de 7000 à 30 000, le copolymère ou homopolymère ayant un rapport de sa composition molaire en acide lactique/acide glycolique de 90/10 à 100/0, puis en soumettant ladite émulsion d'eau dans l'huile à la microencapsulation.

2. Microcapsule selon la revendication 1, dans laquelle ladite émulsion d'eau dans l'huile est dispersée dans une phase aqueuse, puis l'émulsion ternaire obtenue eau/huile/eau est soumise à une dessiccation dans l'eau.

3. Microcapsule selon la revendication 1, dans laquelle l'analogue de LH-RH est soluble dans l'eau et a un poids moléculaire de 1000 ou plus.

4. Microcapsule selon la revendication 1, dans laquelle ledit analogue de LH-RH est (pyr)Glu-His-Trp-Ser-Tyr-D-Leu-Leu-Arg-ProNHC₂H₅ (TAP-144).

5. Microcapsule selon la revendication 1, dans laquelle ledit homopolymère est un acide polylactique ayant un poids moléculaire moyen pondéral de 7000 à 25 000.

6. Microcapsule selon la revendication 1, dans laquelle ladite phase aqueuse interne contient de 25 à 65% (poids/poids) de ladite LH-RH ou de l'analogue.

7. Microcapsule selon la revendication 1, dans laquelle ladite phase aqueuse interne contient de 35 à 60% (poids/poids) de ladite LH-RH ou de l'analogue.

8. Procédé de préparation d'une microcapsule conçue pour une libération d'ordre zéro de l'hormone libérant l'hormone lutéinisante (LH-RH) ou d'un analogue de celle-ci pendant un laps de temps d'au moins deux mois, selon lequel on prépare une émulsion d'eau dans l'huile comprenant une phase aqueuse inerte contenant de 20 à 70% (poids/poids) de ladite LH-RH ou d'un analogue en l'absence d'un agent de rétention du médicament, et une phase huileuse contenant un copolymère ou un homopolymère ayant un poids moléculaire moyen pondéral de 7000 à 30 000, le copolymère ou homopolymère ayant un rapport de sa composition molaire en acide lactique/acide glycolique de 90/10 à 100/0, puis on soumet ladite émulsion d'eau dans l'huile à une dessiccation dans l'eau ou a une méthode de séparation de phases.

9. Procédé selon la revendication 8, dans lequel ladite émulsion d'eau dans l'huile est dispersée dans une phase aqueuse, puis l'émulsion ternaire obtenue eau/huile/eau est soumise a une dessiccation dans l'eau.

10. Procédé selon la revendication 8, dans lequel ladite émulsion d'eau dans l'huile est dispersée dans une phase aqueuse contenant de l'alcool polyvinylique comme agent émulsifiant.

11. Procédé selon la revendication 8, dans lequel ledit analogue de LH-RH est soluble dans l'eau et a un poids moléculaire moyen de 1000 ou plus.

12. Procédé selon la revendication 8, dans lequel ledit analogue de LH-RH est (pyr)Glu-His-Trp-Ser-Tyr-D-Leu-Leu-Arg-ProNHC₂H₅ (TAP-144).

13. Procédé selon la revendication 8, dans lequel ledit homopolymère est un acide polylactique ayant un poids moléculaire moyen pondéral de 7000 à 25 000.

14. Procédé selon la revendication 8, dans lequel ladite phase aqueuse interne contient de 25 à 65% (poids/poids) de ladite LH-RH ou de l'analogue.

15. Procédé selon la revendication 8, dans lequel ladite phase aqueuse interne contient de 35 à 60% (poids/poids) de ladite LH-RH ou de l'analogue.
